# EUROPEAN PATENT APPLICATION

(11) **EP 4 467 560 A1**
(43) Date of publication of application: **27.11.2024**
(21) Application number: 22927447.7
(22) Date of filing: 24.10.2022
(51) Int. Cl.: C07K 7/08, C07K 14/435, A61K 38/00, A61P 17/00, A61K 8/64, A23L 33/18

(54) **PEPTIDE CAPABLE OF PREVENTING OR TREATING ATOPIC DERMATITIS**

(30) Priority: 18.02.2022 KR 20220021469; 06.10.2022 KR 20220127889
(71) Applicant: REMEDI CO., LTD, Incheon 21990 (KR)
(72) Inventor: PARK, Chanho, Incheon 22009 (KR); CHO, Seongmin, Incheon 21986 (KR); SONG, Ee Chan, Incheon 21928 (KR)
(74) Representative: Sander, Rolf
(86) International application number: PCT/KR2022/016269
(87) International publication number: WO 2023/158051

(57) **Abstract**

The present invention relates to a peptide exhibiting an anti-inflammatory function, a fusion compound containing the same, and pharmaceuticals, cosmetics, foods, medical devices, *etc.* containing the same. The present inventors have found that the peptide of SEQ ID NO: 40 and a variant thereof exhibit low cytotoxicity, significantly reduce cellular inflammatory responses induced by lipopolysaccharide, and also exhibit wound healing efficacy.

## Description

### Technical Field

The present invention relates to a peptide exhibiting activity for preventing and/or treating atopic dermatitis, and a composition containing the same, which can be applied to medicines, quasi-drugs, medical devices, and cosmetics.

### Background Art

Atopic dermatitis is a chronic skin disease accompanied by skin dryness, keratinization, and itching, which causes pain to 0.5% to 1% of the total population, especially 5% to 10% of children, and the number of patients is increasing in recent years. In the affected area where atopic dermatitis occurs, there is a significant infiltration of immune-related cells such as macrophages, mast cells, and Th lymphocytes. In patients with atopic dermatitis, the concentration of IgE in the blood increases significantly because the number of Th2 cells increases and IL-4 secreted by these cells stimulates B lymphocytes to promote IgE secretion. Therefore, atopic dermatitis is classified as a Th2-type skin disease related to an abnormality of the immune system. Atopic dermatitis causes significant economic damage along with physical and mental pain to patients, but no specific drug to treat this disease has yet been found. Currently, steroids, antihistamines, *etc.* are used as treatments for atopic dermatitis, but long-term use of these drugs causes serious side effects, and thus there is a need for the development of drugs to replace these drugs.

### Disclosure of the Invention

### Technical Goals

An object of the present invention is to provide a novel peptide for preventing and/or treating atopic dermatitis that is safe and stable to the living body and applicable to cosmetics, pharmaceuticals, *etc.*

Additionally, another object of the present invention is to provide cosmetics, pharmaceutical compositions, medical devices, foods, *etc.* containing the peptide for preventing and/or treating atopic dermatitis.

### Technical Solution

In order to solve the above problems of the present invention, the present inventors selected and testes a number of human-derived candidate peptides, and as a result, they have found that contiguous peptides consisting of 5 or more and 31 or less selected from 31 amino acids, 5'-DVENNSTLEKHSGKRRKKRRHRSKVNGLQRG-3' (SEQ ID NO: 40), which is derived from human SP100 protein from position 519 to position 549, show low cytotoxicity and significantly reduce the cellular inflammatory response induced by polysaccharide, as well as wound healing efficacy and improved efficacy of atopic dermatitis . The activity of the molecule by a mutation is not entirely altered.

Among these peptides, the present inventors named the peptide consisting of 15 amino acids of LEKHSGKRRKKRRHR (SEQ ID NO: 1; a peptide consisting of amino acids from position 526 to position 540 of human SP100 protein) as "RMSP1" or "RMSP1 peptide" hereinafter; named the representative 12 sequences among the peptides consisting of different contiguous amino acids as RMSP1-1 to RMSP1-12, respectively, and tested peptides of various lengths and sequences as to whether they have the effect of reducing or treating cellular inflammatory responses, wound healing efficacy, and efficacy of improving atopic dermatitis.

Additionally, the peptide according to the present invention may be modified by phosphorylation, sulfuration, acrylation, glycosylation, methylation, farnesylation, *etc.*

The peptide or fragment thereof of the present invention may form a conjugate conjugated with a biocompatible polymer or fatty acid. The biocompatible polymer may be at least one selected from the group consisting of starch, dextran, chitosan, glycol chitosan, pullulan, chondroitin sulfate, hyaluronic acid, pectin, polylactic acid (PLA), polyglycolide (PGA), polycaprolactone (PCL), a poly(caprolactone-lactide) random copolymer (PCLA), *etc.,* but is not limited thereto. Additionally, the fatty acid may be one or more selected from the group consisting of hexanoic acid, caprylic acid, capric acid, lauric acid, palmitic acid, stearic acid, and cholesterol, but is not limited thereto.

Unless defined otherwise, all terms used in the present invention, including technical or scientific terms, have the same meaning as commonly understood by one of ordinary skill in the art to which the present invention pertains.

### Advantageous Effects

The anti-inflammatory peptide of the present invention exhibited low cytotoxicity and significantly reduced the cellular inflammatory response induced by polysaccharide, while exhibiting wound healing efficacy and treatment efficacy of atopic dermatitis.

Therefore, the peptide for preventing and/or treating atopic dermatitis of the present invention and the compound containing the same can be used as medicines, cosmetics, food compositions for preventing or alleviating inflammation, wound healing agents, fillers, complex fillers, *etc.*

### Brief Description of Drawings

FIG. 1 shows a predicted secondary structure of the RMSP1 peptide, in which (a) shows the secondary structure of the peptide predicted using the Pep-fold3 program, and (b) shows a graph showing the Pepfold of the peptide.
FIG. 2 shows the purity of the RMSP1 peptide.
FIG. 3 shows the wound healing efficacy of the SP100 peptide, in which (a) shows that the wound induced by performing the scratch assay recovers over time, and (b) shows a graph of the degree of wound closure.
FIG. 4 shows the anti-inflammatory efficacy of RMSP1.
FIG. 5 shows the evaluation result of efficacy of the RMSP1 peptide treating atopy, in which (a) shows the images of an untreated control group (Con), a group where atopy is induced by oxazolone treatment (Oxa), and a group where atopy is induced by oxazolone treatment and then treated with RMSP1 (RMSP1), (b) shows the weight of ear biopsies of the same area taken from each group, and (c) shows the images of stained a longitudinal section of the ear tissue and a graph of the thickness of the dermis measured using Image J.
FIG. 6 shows the result of genetic analysis of ear tissue of an animal model with atopy.
FIG. 7 shows the evaluation result of anti-inflammatory efficacy by treating RAW264.7 macrophages with mutants of RMSP1 in order to confirm whether the efficacy is maintained or improved through mutation, substitution, and deletion of amino acids of the RMSP1 peptide. As a result, it was confirmed that the anti-inflammatory effect was maintained and the anti-inflammatory effect was increased in some variants.
FIG. 8 shows the comparison result of efficacies after attaching cell penetrating peptide AD or MR to the N-terminal part of RMSP1 and proceeding with acetylation or palmitoylation. As a result, palmitoylated pal-RMSP1 showed the highest anti-inflammatory efficacy, and AD-RMSP1 attached with AD cell penetrating peptide showed higher inhibitory efficacy than MR-RMSP1.
FIG. 9 confirms the anti-inflammatory efficacy of the peptide, which was obtained by palmitoylation of the N-terminal position of RMSP1, using skin cells. After inducing the expression of inflammatory genes in HaCaT cells by treating them with TNF-α and IFN-γ, the anti-inflammatory efficacy of pal-RMSP1 was tested, and as a result, it was confirmed that inflammatory genes were suppressed in TNF-α, IL-8, CCL-5, CCL-17, and CCL-17 22 according to concentration.

### Description of Exemplary Embodiments

The definitions of key terms used in the description and claims of the present invention are as follows.

"Amino acid" and "amino acid residue" refer to natural amino acids, non-natural amino acids, or modified amino acids. Unless otherwise stated, all references to amino acids, either generically or specifically by designation, include reference to both the D and L stereoisomers (where the structure permits such stereoisomeric forms). Natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr) and valine (Val). Non-natural amino acids include modified amino acid residues that have been chemically modified on the N-terminal amino group or side chain group, or reversibly or irreversibly chemically blocked, for example, N-methylated D and L amino acids or side chain functionalities that have been chemically modified with another functional group.

As used herein, "conservative substitution" refers to a modification of a cargo molecule transport domain that includes substituting one or more amino acids with amino acids having similar biochemical properties that do not cause loss of biological or biochemical functions of the cargo molecule transport domain.

As used herein, "conservative amino acid substitution" refers to a substitution in which an amino acid residue is substituted with an amino acid residue having a similar side chain. Classes of amino acid residues with similar side chains have been defined in the art and are well known. These classes include amino acids with basic side chains (*e.g.*, lysine, arginine, and histidine), amino acids with acidic side chains (*e.g.*, aspartic acid and glutamic acid), and amino acids with uncharged polar side chains (*e.g.*, glycine, asparagine, glutamine, serine, threonine, tyrosine, and cysteine), amino acids with non-polar side chains (*e.g.*, alanine, valine, leucine, isoleucine, proline, phenylalanine, methionine, and tryptophan), amino acids with beta-branched side chains (*e.g.*, threonine, valine, and isoleucine), and amino acids with aromatic side chains (*e.g*., tyrosine, phenylalanine, tryptophan, and histidine).

Other terms described in this specification, claims, and drawings are used in the meaning generally used by those of ordinary skill in the art to which the present invention pertains, unless otherwise specified.

The present invention relates to a peptide for preventing or treating atopic dermatitis selected from:
a) a peptide consisting of SEQ ID NO: 40;
b) a deleted peptide of SEQ ID NO: 40 consisting of 5 to 30 amino acids, in which in the peptide consisting of SEQ ID NO: 40, 1 to 13 contiguous amino acids from the N terminus are deleted and/or 1 to 20 contiguous amino acids from the C terminus are deleted;
c) a substituted peptide of SEQ ID NO: 40, in which any one or more amino acids in the peptide consisting of SEQ ID NO: 40 of a) above and the deleted peptide of SEQ ID NO: 40 of b) above are substituted with conservative amino acids; and
d) a homologous peptide that exhibits at least 80%, preferably at least 85%, more preferably at least 90%, and most preferably at least 95% sequence homology with the peptide selected from a), b) and c) above, and a homologous peptide exhibiting atopic dermatitis prevention or treatment activity of at least 70%, preferably at least 75%, more preferably at least 80%, more preferably at least 85%, and even more preferably at least 90% of the activity of the selected peptide.

Additionally, the present invention relates to a peptide for preventing or treating atopic dermatitis, wherein the conservative amino acid substitution in c) is in which the arginine residue position is independently substituted with a lysine residue and/or the lysine residue position is substituted with an arginine residue.

Additionally, the present invention provides a peptide for preventing or treating atopic dermatitis, where the deletion peptide of SEQ ID NO: 40 of b) is not limited to the sequence below, but is preferably any one selected from SEQ ID NO: 1 to SEQ ID NO: 13.

Additionally, the present invention relates to a peptide for preventing or improving atopic dermatitis, which is characterized in that the peptide for preventing or improving atopic dermatitis possesses wound healing efficacy.

Additionally, the present invention relates to a peptide for preventing or treating atopic dermatitis, wherein any one of the above peptides for preventing or treating atopic dermatitis is modified by phosphorylation, sulfuration, acrylation, glycosylation, methylation or farnesylation.

Additionally, the present invention relates to a fusion compound including a peptide for preventing or improving atopic dermatitis, in which a functional molecule is bound to at least one of the N-terminus and C-terminus of the peptide for preventing or treating atopic dermatitis.

Additionally, the present invention relates to a fusion compound including a peptide for preventing or improving atopic dermatitis, which is characterized in that the functional molecules are therapeutic peptides including hormones, growth factors, neurotransmitters, and ion channel ligands, or therapeutic proteins such as various enzymes including superoxide dismutase, albumin, and antibodies.

Additionally, the present invention relates to a fusion compound including a peptide for preventing or improving atopic dermatitis, which is characterized in that the functional molecule is a molecule that exhibits, for example, an antioxidant function as in peroxyredoxin, or an anti-inflammatory function as in interleukin, or a wound healing function.

Additionally, the present invention relates to a fusion compound including a peptide for preventing or treating atopic dermatitis, wherein the functional molecule is a therapeutic peptide or a therapeutic protein.

Additionally, the present invention relates to a fusion compound including a peptide for preventing or treating atopic dermatitis, wherein the functional molecule is a molecule exhibiting antioxidant, anti-inflammatory or wound healing functions.

Additionally, the present invention relates to a pharmaceutical composition for preventing or treating skin inflammation, comprising the peptide for preventing or treating atopic dermatitis or a fusion compound containing the peptide for preventing or treating atopic dermatitis.

Additionally, the present invention relates to a pharmaceutical composition for preventing or treating diseases in which the skin inflammation is atopic dermatitis.

Additionally, the present invention relates to a cosmetic composition comprising the peptide for preventing or treating atopic dermatitis or a fusion compound containing the peptide for preventing or treating atopic dermatitis.

Additionally, the present invention relates to a food composition containing the peptide for preventing or treating atopic dermatitis or a fusion compound containing the peptide for preventing or treating atopic dermatitis.

Additionally, the present invention relates to a medical device selected from among wound dressings, fillers, or composite fillers containing the peptide for preventing or treating atopic dermatitis or a fusion compound containing the peptide for preventing or treating atopic dermatitis.

Additionally, the present invention relates to a pharmaceutical composition for preventing or treating a disease including a peptide for preventing or improving atopic dermatitis or a fusion compound, in which a functional molecule is bound to at least one of the N-terminus and C-terminus of the peptide for preventing or improving atopic dermatitis. The disease may include, for example, skin inflammation such as atopy, but is not limited thereto. In particular, the pharmaceutical composition of the present invention is useful for the prevention and treatment of wounds and inflammation caused by trauma such as dermatitis, particularly atopic dermatitis, skin cuts, wounds, and abrasions.

Additionally, the present invention relates to a cosmetic composition including a peptide for preventing or improving atopic dermatitis or a fusion compound, in which a functional molecule is bound to at least one of the N-terminus and C-terminus of the peptide for preventing or improving atopic dermatitis. According to an exemplary embodiment, the cosmetic composition can alleviate inflammation and improve wounds when a wound occurs on the skin, and has an effect of preventing and improving atopic dermatitis. Cosmetics of the present invention may include color cosmetics (e.g., foundations, lipsticks, eye shadows, *etc.)* in addition to basic cosmetics (e.g., toners, creams, essences, water-in-water or water-in-oil emulsions, ointments, etc.).

Additionally, the present invention relates to a food composition, which exhibits the function of preventing or improving atopic dermatitis and/or the function of wound healing, including a peptide for preventing or improving atopic dermatitis or a fusion compound, in which a functional molecule is bound to at least one of the N-terminus and C-terminus of the peptide for preventing or improving atopic dermatitis.

Besides, the present invention relates to a medical device, which includes the one or more of the peptides for preventing or improving atopic dermatitis or a fusion compound, in which a functional molecule is bound to at least one of the N-terminus and C-terminus of the peptide for preventing or improving atopic dermatitis, and which is selected from a wound dressing, a filler, and a complex filler.

As used herein, the term "conservative substitution" refers to modification of a cargo molecule transduction domain including substituting one or more amino acids by amino acids having similar biological or biochemical properties that do not cause loss of the biological or biochemical functions of the cargo molecule transduction domain.

As used herein, the term "conservative amino acid substitution" refers to a substitution to replace an amino acid residue by an amino acid residue having a similar side chain. Classes of the amino acid residue having a similar side chain are defined and well-known in the art. Such classes include amino acids with basic side chains (for example, lysine, arginine, histidine), amino acids with acidic side chains (for example, aspartic acid, glutamic acid), amino acids with uncharged polar side chains (for example, glycine, asparagine, glutamine, serine, threonine, tyrosine, cysteine), amino acids having non-polar side chains (for example, alanine, valine, leucine, isoleucine, proline, phenylalanine, methionine, tryptophan), amino acids having beta-branched side chains (for example, threonine, valine, isoleucine) and amino acids having aromatic side chains (for example, tyrosine, phenylalanine, tryptophan, and histidine).

The peptide for preventing or improving atopic dermatitis according to the present invention is interpreted as including variants in which one or more amino acids in the RMSP1 peptide are modified due to substitution, deletion, and/or addition.

Additionally, the peptide variant for preventing or improving atopic dermatitis according to the present invention is interpreted as also including cargo molecule transport domain variants or fragments thereof, which have substantially the same function and/or effect as the cargo molecule transport domain according to the present invention, and amino acid sequence homology of 80% or 85% or more, preferably 90% or more, more preferably 95% or more, to the cargo molecule transport domain.

Additionally, it is expected that the peptide for preventing or improving atopic dermatitis of the present invention may still have the activity for preventing or improving atopic dermatitis even if it has a conservative amino acid substitution, amino acid deletion, or amino acid addition.

Additionally, the present invention is characterized in that the functional molecule is selected from proteins, peptides, nucleic acids (*e.g.*, oligonucleotides, polynucleotides, etc.), carbohydrates, lipids, and mixtures of one or more of these.

Additionally, the present invention is characterized in that the chemical bond between the functional molecule and the peptide for preventing or improving atopic dermatitis is a covalent bond or a non-covalent bond, preferably a covalent bond. The chemical bond may be a covalent bond or a non-covalent bond. Non-covalent bonds may include ionic bonds, bonds by electrostatic attraction, bonds by hydrophobic interactions, *etc.* Additionally, the material capable of binding to the anti-inflammatory peptide through ionic bonding or electrostatic attraction may be a charged material such as DNA or RNA.

The peptides for preventing or improving atopic dermatitis of the present invention are not limited to SEQ ID NOS: 1 to 13, but it is clearly stated that representative peptides are shown in Table 1 for convenience of experiments.

Additionally, the pharmaceutical composition including the peptide for preventing or improving atopic dermatitis of the present invention and a fusion compound of the peptide for preventing or improving atopic dermatitis and a functional molecule as active ingredients may be formulated with a carrier commonly accepted in the pharmaceutical field in various forms (*e.g.*, external skin preparations, oral preparations, sprays, patches, injections, *etc.)* by conventional methods. For example, oral compositions may include tablets and gelatin capsules, which, in additional to the active ingredients, may include a diluent (*e.g.*, lactose, dextrose, sucrose, mannitol, sorbitol, cellulose, and/or glycine), a lubricant (*e.g*., silica, talc, stearic acid, magnesium or calcium salts thereof, and/or polyethylene glycol), and the tablets may also include a binder (*e.g.*, magnesium aluminum silicate, starch paste, gelatin, methylcellulose, sodium carboxymethylcellulose, and/or polyvinylpyrrolidone), optionally including a disintegrant (*e.g*., starch, agar, alginic acid or a sodium salt thereof) or boiling mixtures and/or an absorbent, a colorant, a flavor, and a sweetener. Injectable compositions are preferably isotonic aqueous solutions or suspensions, and the compositions mentioned are sterile and/or include an adjuvant (*e.g*., a preservative, a stabilizer, a wetting agent or emulsifying agent, a solution accelerator, salts and/or buffers for regulating osmotic pressure). Additionally, these may include other therapeutically useful materials.

The pharmaceutical preparation prepared in this way may be administered orally or parenterally, that is, intravenous, subcutaneous, intraperitoneal or topically applied, as desired. The dose administered may be divided into a daily dose of 0.0001 mg/kg to 100 mg/kg into one to several times. The administration dose level for a specific patient may vary depending on the patient's weight, age, sex, health condition, administration time, administration method, excretion rate, severity of disease, *etc.*

The term "food" used in the present invention refers to a natural product or processed product including one or more nutrients, and preferably refers to a product that is in a state to eat directly through a certain degree of processing process. As a conventional meaning, it may include all foods, food additives, functional foods, health functional foods, health supplement foods, and beverages. The food composition of the present invention may be prepared in the form of various beverages, chewing gums, teas, cookies, vitamin complexes, health supplements, *etc.,* without limitation. The preferred intake amount of the food composition of the present invention varies depending on the condition, body weight, severity of symptoms, food type, and intake period of the person who consumes the food, and may appropriately be selected. In the food composition of the present invention, it is preferable for the optimal effect that the active ingredients be ingested in an amount of 0.2 mg/kg to 200 mg/kg per day.

Additionally, the pharmaceutical composition for preventing or treating a disease including, as active ingredients, the peptide for preventing or improving atopic dermatitis and the fusion compound in which a functional molecule is bound to the peptide for preventing or improving atopic dermatitis is characterized in that it is an external skin preparation for treating skin inflammation. An application agent, which includes, as an active ingredient, the peptide for preventing or improving atopic dermatitis, or the fusion compound in which a functional molecule is bound is added, can easily be prepared in any form according to a conventional preparation method. As an example, in preparing a cream-type application agent, the anti-inflammatory composition of the present invention is included in a general oil-in-water (O/W) or water-in-oil (W/O) cream base, and while adding fragrances, chelating agents, pigments, antioxidants, and preservatives thereto as necessary, synthetic or natural materials (*e.g.*, proteins, minerals, vitamins, *etc*.) may be used in combination for the purpose of improving physical properties.

Other terms described in this specification, claims, and drawings are used in the meaning generally used by those of ordinary skill in the art to which the present invention pertains, unless otherwise specified.

**[Table 1]**

| Name | Amino Acid Sequence | Site of Human SP100 | Length | SEQ ID NO |
|---|---|---|---|---|
| RMSP1 | LEKHSGKRRKKRRHR | 526-540 | 15mer | 1 |
| RMSP1-1 | GKRRKKRRHR | 531-540 | 10mer | 2 |
| RMSP1-2 | HSGKRRKKRRHR | 529-540 | 12mer | 3 |
| RMSP1-3 | GKRRKKRRHRSK | 531-542 | 12mer | 4 |
| RMSP1-4 | LEKHSGKRRKKR | 526-537 | 12mer | 5 |
| RMSP1-5 | LEKHSGKRRKKRRHRSKVN | 526-544 | 19mer | 6 |
| RMSP1-6 | NSTLEKHSGKRRKKRRHR | 523-540 | 18mer | 7 |
| RMSP1-7 | DVENNSTLEKHSGKRRKKRRHR | 519-540 | 22mer | 8 |
| RMSP1-8 | GKRRKKRRHRSKVNGLQRG | 531-549 | 18mer | 9 |
| RMSP1-9 | DVENNSTLEKHS | 519-530 | 12mer | 10 |
| RMSP1-10 | DVENNSTLEKHSGKRRKKR | 519-537 | 19mer | 11 |
| RMSP1-11 | LEKHS | 526-530 | 5mer | 12 |
| RMSP1-12 | LEKHSGKRR | 526-534 | 9mer | 13 |

### Description of Exemplary Embodiments

In the following, the configuration of the present invention will be described in more detail with reference to specific embodiments. However, it is apparent to those skilled in the art that the scope of the present invention is not limited only to the description of these specific embodiments.

### 1. Cell culture

HaCaT and RAW264.7 cells were cultured by adding 10% fetal bovine serum (FBS) and an antibiotic solution (100 units/mL penicillin, 100 µg/mL streptomycin) to DMEM medium. The cells were cultured where 37°C, 95% humidity, and 5% CO₂ conditions were maintained, and when the cells reached 70% to 80% adherence to the culture dish, the cells were treated with trypsin-EDTA and subcultured. HaCaT cells were distributed by the laboratory of Professor Tae-Yoon KIM, College of Medicine, Catholic University of Korea and cultured, and RAW264.7 cells were distributed by the laboratory of Professor Sang-Beom KIM, College of Pharmacy, Sahmyook University and cultured from.

### 2. Synthesis of RMSP1 peptide, etc. for preventing or treating atopic dermatitis

Peptides for preventing or treating atopic dermatitis of the present invention, such as RMSP1, were synthesized using a standard Fmoc-SPPS (Solid Phase Peptide Synthesis) protocol using 2-chlorotritylchloride resin and amino acids. The peptides were then cleaved from the resin and side chain protecting groups were removed from the amino acids using TFA acid, water, and triisopropylsilane. Ether was added for precipitation and the peptides were purified using reverse phase HPLC using a C18 column. Elution was performed with a water-acetonitrile linear gradient (0-75% (v/v) of acetonitrile) including 0.1% (v/v) TFA. To remove TFA, the peptides were dissolved in water and 100 mM HCl was added to prepare 8 mM HCl. The solution was incubated at room temperature for 5 minutes, frozen in liquid nitrogen, lyophilized overnight to remove all liquid, the lyophilized powder was redissolved in a HCl solution, frozen again and lyophilized overnight. Lyophilization was repeated 3 times for redissolution. After the final lyophilization step, the peptides were redissolved in water and the molecular weight of the purified peptides was confirmed using LC/MS.

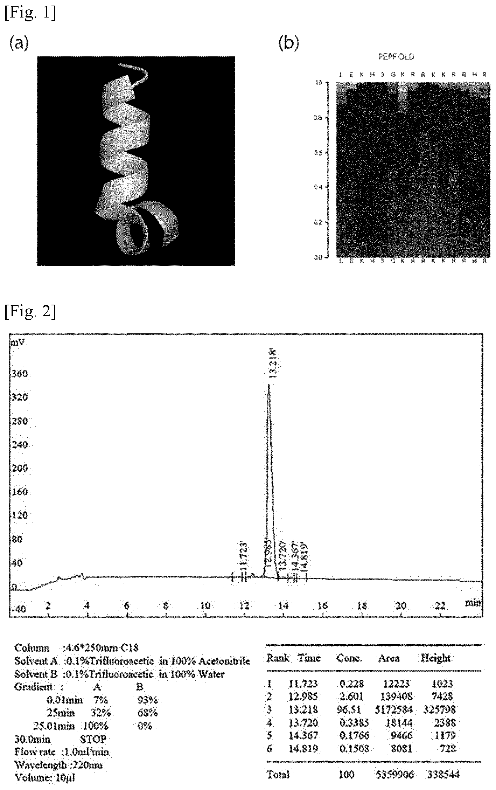

The results are shown in FIG. 2.

### 3. RMSP1 peptide secondary structure prediction

In order to predict the peptide structure of RMSP1, the PEP-FOLD3 *De novo* peptide structure prediction program was used. The model predicted with the program by writing the amino acid sequence of RMSP1 was imaged as a two-dimensional predicted structure with the PyMOL 2.4 program [FIG. 1(a)]. In the prediction profile used for secondary structure prediction analysis, red represents a spiral, green represents an extension, and blue represents a coil, through which a secondary prediction model was derived [FIG. 1(b)],

### 4. Assessment of inflammation inhibition

In order to evaluate the anti-inflammatory efficacy of the peptides for preventing or treating atopic dermatitis of the present invention in RAW264.7 macrophages, RAW264.7 cells in the amount of 5 × 10⁴ were attached to a 24-well plate 12 hours in advance. Then, the cells were cultured in serum-free medium for 1 hour and then treated with 10 µM peptide for 2 hours. Thereafter, each well was treated with 100 ng/mL of lipopolysaccharide (LPS), and after 4 hours, the medium was collected, centrifuged at 1,000 g for 10 minutes, and IL-6 and TNF-α were detected.

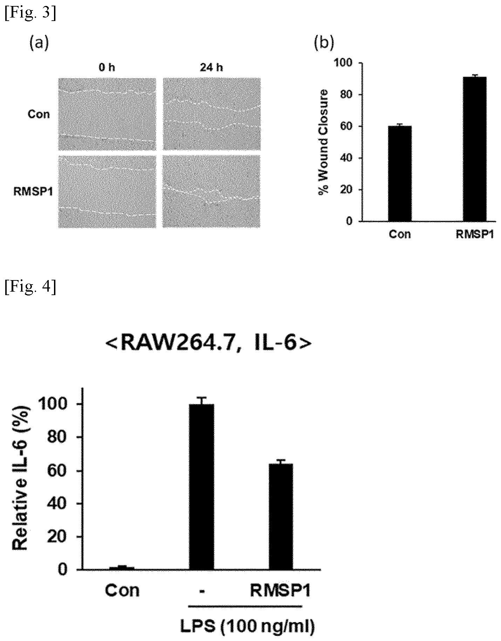

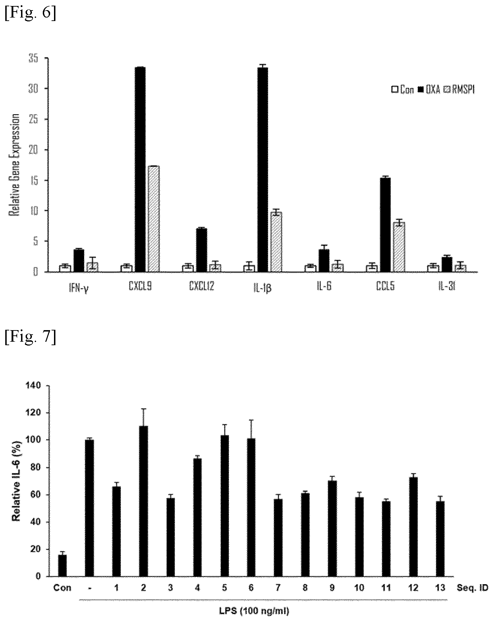

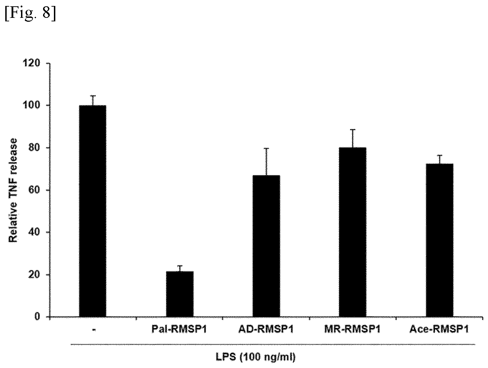

IL-6 analysis results are shown in FIGS. 4 and 7, and TNF-α analysis results are shown in FIG. 8.

### 5. Cell wound healing experiment

In order to confirm the wound healing efficacy of the peptides for preventing or treating atopic dermatitis of the present invention in HaCaT skin cells, a scratch assay was performed. 2 × 10 ⁵ HaCaT cells were attached to each well using a 24-well plate, and then wounds were induced in the mice using a 200 µL pipette tip. Then, the cells were washed twice using a serum-free medium, and the peptides were treated in a serum-free medium at different concentrations. Cell images were obtained using a microscope after 0 and 24 hours, respectively, and the width of the wounds was measured and analyzed using the ImageJ program.

The results of confirming the wound healing efficacy using skin cells are shown in FIG. 3. FIG. 3(a) is an image showing the wound healing efficacy over time of the RMSP1 peptide for preventing or treating atopic dermatitis of the present invention, and FIG. 3(b) shows the graphed result of the degree of wound closure.

### 6. Atopic mouse animal model

In an atopic mouse animal model, the atopic treatment efficacy was evaluated using the RMSP1 peptide for preventing or treating atopic dermatitis of the present invention. After dissolving 2% oxazolone in a solvent in acetone:olive oil = 4:1 ratio, the inner and outer ears were sensitized with 10 µL. After 7 days, 10 µL of 0.5% oxazolone was applied to the inner and outer ears. In the case of the peptide for preventing or treating atopic dermatitis of the present invention, from 1 day after sensitization, 10 µg each was applied to the surface of the outer ear every day for the indicated days.

The mice sacrificed on day 10 and the weight of the ear tissue was measured using a 5 mm biopsy punch, and then the tissue was fixed with a 4% paraformaldehyde (PFA) solution to prepare paraffin sections with a size of 5 µm. Then, a representative image was obtained through H&E staining, and then the thickness of the dermal layer was measured using Caseviewer.

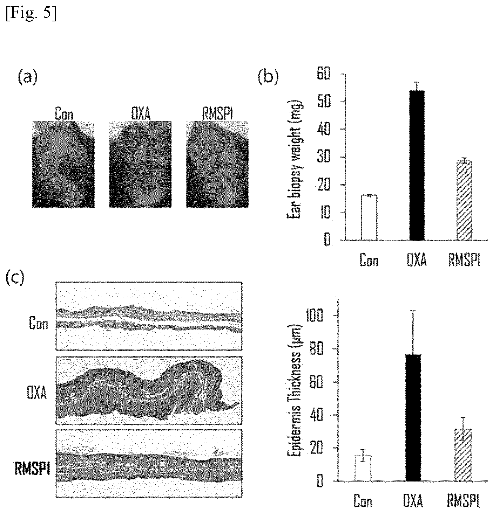

Atopic dermatitis was induced by applying oxazolone to the ear of each mouse in the same manner as above, and then treated with the peptide of the present invention for preventing or treating atopic dermatitis is shown in FIG. 5 (a); FIG. 5(b) shows the result of measuring the weight of the ear of each mouse for the same area; and FIG. 5(c) is a graph showing the thickness of the dermis using the image of a stained longitudinal section of the ear tissue and imageJ.

### 7. Gene analysis of atopic animal model

In the case of qPCR, the ear tissue of each atopic animal model obtained using a 5mm biopsy punch was used, in which RNA was obtained using the Rneasy kit, and cDNA synthesis was performed. Then, the expression level of each gene was measured using the primers listed in Table 2 below and SYBR green reagent. The relative expression level was confirmed using β-actin, which is a housekeeping gene. The results are shown in FIG. 6.

**[Table 2]**

| **Mouse Gene** | **Forward primer** | **Reverse primer** |
|---|---|---|
| **IFN-γ** | 5'-AAGTGGCATAGATGTGGAAG-3' (SEQ ID NO: 14) | 5'-GAATGCATCCTTTTTCGCCT-3' (SEQ ID NO: 15) |
| **CXCL9** | 5'-CTTGAGCCTAGTCGTGATAAC-3' (SEQ ID NO: 16) | 5'-CCAGCTTGGTGAGGTCTATC-3' (SEQ ID NO: 17) |
| **CXCL12** | 5'-CAGAGCCAACGTCAAGCA-3' (SEQ ID NO: 18) | 5'-AGGTACTCTTGGATCCAC-3' (SEQ ID NO: 19) |
| **IL-1β** | 5'-TTGAAGAAGAGCCCATCCTCTG-3' (SEQ ID NO: 20) | 5'-GATCCACACTCTCCAGCTGC-3' (SEQ ID NO: 21) |
| **IL-6** | 5'-ACCACGGCCTTCCCTACTTC-3' (SEQ ID NO: 22) | 5'-CTCATTTCCACGATTTCCCAG-3' (SEQ ID NO: 23) |
| **CCL-5** | 5'-TGCCCACGTCAAGGAGTATTTC-3' (SEQ ID NO: 24) | 5'-AACCCACTTCTTCTCTGGGTTG-3' (SEQ ID NO: 25) |
| **IL-31** | 5'-ATACAGCTGCCGTGTTTCAG-3' (SEQ ID NO: 26) | 5'-AGCCATCTTATCACCCAAGAA-3' (SEQ ID NO: 27) |
| **β-actin** | 5'-ACCAACTGGGACGACATGGAGAA-3' (SEQ ID NO: 28) | 5'-GTGGTGGTGAAGCTGTAGCC-3' (SEQ ID NO: 29) |

### 8. Confirmation of anti-inflammatory effect of Pal-RMSP1

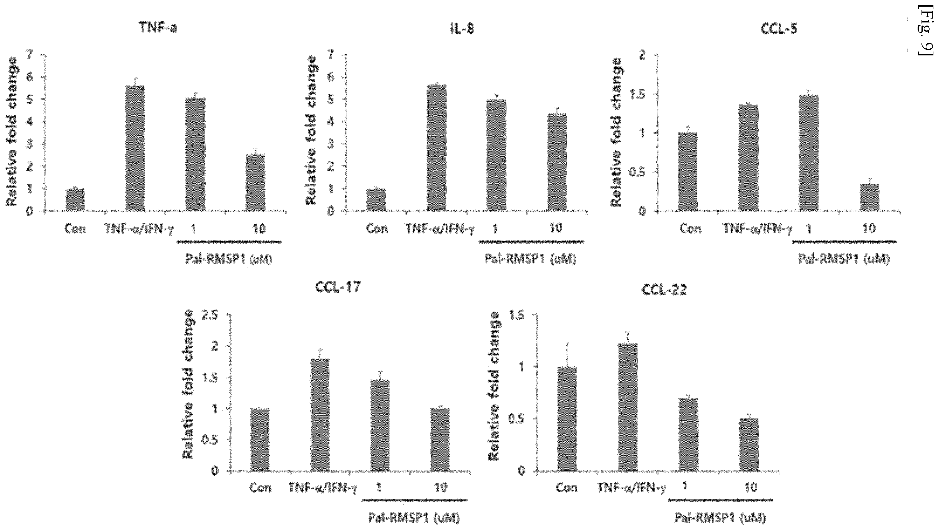

Inflammatory cytokine gene expression was analyzed in HaCaT human skin keratinocytes. HaCaT cells were attached to each well of a 6-well plate at 3 × 10⁵ and cultured for 24 hours. Then, the cells were washed using a serum-free medium, cultured in the serum-free medium for 16 hours, and treated with 1 µM and 10 µM peptide for 2 hours, respectively. Each well was treated with 10 mg/mL of TNF-α and IFN-γ and cultured for 6 hours. The cells were washed with PBS, and RNA was obtained using the EZ Total RNA Miniprep Kit (Enzynomics), and cDNA synthesis was performed using the TOPscript cDNA Synthesis Kit (Enzynomics). The expression level of each gene was measured using the primers shown in Table 3 and PowerUp SYBR Green Master Mix (AppliedBiosystems) reagent. The relative expression level was evaluated using GADPH, which is a housekeeping gene. As a result, the anti-inflammatory effect of the peptides obtained by palmitoylation of the N-terminal position of RMSP1 using skin cells was confirmed. In TNF-α, IL-8, CCL-5, CCL-17, and CCL-22, it was confirmed that inflammatory genes were inhibited according to concentrations. The results are shown in FIG. 9.

**[Table 3]**

| Gene | Forward primer | Reverse primer |
|---|---|---|
| TNF-α | 5'-CTG AAA GCA TGA TCC GGG AC-3' (SEQ ID NO: 30) | 5'-TTAGAG AGA GGT CCC TGG GG-3' (SEQ ID NO: 31) |
| IL-8 | 5'-CTC TGT GTG AAG GTG CAG TT-3' (SEQ ID NO: 32) | 5'-GGG GTG GAA AGG TTT GGA GTA-3' (SEQ ID NO: 33) |
| CCL-5 | 5'-CAG TCG TCT TTG TCA CCC GA-3' (SEQ ID NO: 34) | 5'-CGG GTG GGG TAG GAT AGT GA- 3' (SEQ ID NO: 35) |
| CCL-17 | 5'-ATG GCC CCA CTG AAG ATG C-3' (SEQ ID NO: 36) | 5'-AAA ACG ATG GCA TCC CTG GA- 3' (SEQ ID NO: 37) |
| CCL-22 | 5'-ATC GCC TAC AGA CTG CAC TC-3' (SEQ ID NO: 38) | 5'-TCG GCA CAG ATC TCC TTA TCC- 3' (SEQ ID NO: 39) |

### Industrial Applicability

The anti-inflammatory peptide of the present invention exhibits low cytotoxicity, significantly reduces cellular inflammatory responses induced by lipopolysaccharide, and also exhibits wound healing efficacy and treatment efficacy on atopic dermatitis; therefore, the anti-inflammatory peptide of the present invention can be used in medicines and cosmetics for applying to inflammation including skin inflammation (*e.g*., atopic dermatitis, *etc*.), food compositions for preventing or alleviating inflammation, wound healing agents, fillers, complex fillers, *etc.*

### Sequence List Free Text

Electronic file attatched.

## Claims

1. A peptide for preventing or treating atopic dermatitis selected from:
a) a peptide consisting of SEQ ID NO: 40;
b) a deleted peptide of SEQ ID NO: 40 consisting of 5 to 30 amino acids, in which in the peptide consisting of SEQ ID NO: 40, 1 to 13 contiguous amino acids from the N terminus are deleted and/or 1 to 20 contiguous amino acids from the C terminus are deleted;
c) a substituted peptide of SEQ ID NO: 40, in which any one or more amino acids in the peptide consisting of SEQ ID NO: 40 of a) above and the deleted peptide of SEQ ID NO: 40 of b) above are substituted with conservative amino acids; and
d) a homologous peptide that exhibits at least 80% sequence homology with the peptide selected from a), b) and c) above, a homologous peptide exhibiting atopic dermatitis prevention or treatment activity of at least 70% of the activity of the selected peptides.

2. The peptide of claim 1, wherein the substation with conservative amino acid is one in which an amino acid residue at an arginine residue position is independently substituted with a lysine residue and/or an amino acid residue at a lysine residue is independently substituted with an arginine residue.

3. The peptide of claim 1, wherein the deleted peptide of SEQ ID NO: 40 of b) above is any one selected from the peptides of SEQ ID NO: 1 to SEQ ID NO: 13.

4. The peptide for preventing or treating atopic dermatitis selected from claims 1 to 3 is a peptide for preventing or treating atopic dermatitis, which is modified by phosphorylation, sulfuration, acrylation, glycosylation, methylation or farnesylation.

5. A fusion compound comprising a peptide for preventing or treating atopic dermatitis, wherein a functional molecule is bound to one or more of the N-terminus and the C-terminus of the peptide for preventing or treating atopic dermatitis selected from claims 1 to 3.

6. A fusion compound comprising a peptide for preventing or treating atopic dermatitis, in which a functional molecule is bound to any one or more sites of the N-terminus and the C-terminus of the peptide of claim 4.

7. The fusion compound of claim 5, wherein the functional molecule is a therapeutic peptide or therapeutic protein.

8. The fusion compound of claim 6, wherein the functional molecule is a therapeutic peptide or therapeutic protein.

9. The fusion compound of claim 7, wherein the functional molecule is a molecule that exhibits a function of antioxidation, anti-inflammation, or wound healing.

10. The fusion compound of claim 8, wherein the functional molecule is a molecule that exhibits a function of antioxidation, anti-inflammation, or wound healing.

11. A pharmaceutical composition for preventing or treating skin inflammation comprising a fusion compound comprising a peptide for preventing or treating atopic dermatitis selected from claims 1 to 4 or a peptide for preventing or treating atopic dermatitis selected from claims 5 to 10.

12. A pharmaceutical composition for preventing or treating diseases, wherein the skin inflammation of claim 11 is atopic dermatitis.

13. A cosmetic composition for preventing or treating skin inflammation comprising a fusion compound comprising a peptide for preventing or treating atopic dermatitis selected from claims 1 to 4 or a peptide for preventing or treating atopic dermatitis selected from claims 5 to 10.

14. A food composition for preventing or treating skin inflammation comprising a fusion compound comprising a peptide for preventing or treating atopic dermatitis selected from claims 1 to 4 or a peptide for preventing or treating atopic dermatitis selected from claims 5 to 10.

15. A medical device, which comprises a fusion compound comprising a peptide for preventing or treating atopic dermatitis selected from claims 1 to 4 or a peptide for preventing or treating atopic dermatitis selected from claims 5 to 10, and selected from wound dressing, filler, or composite filler.
